(19) **Europäisches Patentamt**
**European Patent Office**
**Office européen des brevets**

(11) **EP 4 252 640 A1**

(12) **EUROPEAN PATENT APPLICATION**

(43) Date of publication:
04.10.2023 Bulletin 2023/40

(21) Application number: 22165745.5

(22) Date of filing: 31.03.2022

(51) International Patent Classification (IPC):
**A61B 5/06** (2006.01)       **A61B 5/287** (2021.01)
**A61B 5/00** (2006.01)       A61B 34/20 (2016.01)

(52) Cooperative Patent Classification (CPC):
**A61B 5/065; A61B 5/287; A61B 5/6852;**
A61B 2034/2053; A61B 2560/0223;
A61B 2560/0238

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **KAHLMAN, Josephus Arnoldus Johannes Maria
Eindhoven (NL)**
• **MEGENS, Mischa
Eindhoven (NL)**

(74) Representative: **Philips Intellectual Property &
Standards
High Tech Campus 52
5656 AG Eindhoven (NL)**

(54) **DETERMINING THE DISTANCE BETWEEN DEVICE ELECTRODES**

(57)     A mechanism for determining a distance between electrodes on an interventional device. The electrical responses of the electrodes to electric current applied to calibration electrodes are used to derive or determine the inter-electrode distance(s).

FIG. 2

EP 4 252 640 A1

**Description**

FIELD OF THE INVENTION

The present invention relates to the field of interventional devices, and in particular, to approaches for determining the distance between device electrodes carried by an interventional device.

BACKGROUND OF THE INVENTION

There is an increasing interest in the use of interventional devices. An interventional device is a device that is designed to be positioned or positionable within a subject or individual. An interventional device comprises one or more device electrodes, which can be used for a variety of use case scenarios.

One advantageous use case scenario is in the monitoring of the location of the interventional device. In such scenarios, a mapping or tracking system can be configured to track the location of any electrodes carried by the interventional device, e.g. by monitoring the response of the electrode(s) to induced (crossing) electric fields.

One example of a suitable system that achieves this goal is the EPD Solutions navigation system provided by Philips®, named the KODEX-EPD® system. This system exploits (di)electric field sensing for 3D localization, allowing radiation-free navigation in an anatomical cavity, e.g. inside the heart.

Known mapping techniques, such as those employed by the KODEX-EPD system, make use of crossing electrical fields between three pairs of external electrodes that represent the x-, y-, and z-dimension. Voltages at device electrodes are sampled, and a real-time voltage-to-position function or mapping function is formed. The mapping function relates a voltage of an electrode to a position in a predefined co-ordinate system. This mapping function is continuously updated based on the measurements coming in. Once sufficiently updated, the mapping function enables the system to reliably track or monitor the spatial movement of the interventional device. If the interventional device is moved within an anatomical cavity or structure, it is possible to generate an anatomical model of the anatomical cavity or structure based on the monitored locations (as it can be assumed that the interventional device moves only within the cavity itself).

Typically, one requirement for generating an accurate mapping function is knowledge of the distance between the device electrodes or "inter-electrode distance". This information is necessary, because electric fields induced within a subject are typically inhomogeneous, meaning that there may not be a continuous proportional relationship between differences in voltage between two device electrodes and the geometrical distance between said device electrodes.

There is an increasing desire to improve the flexibility of mapping or tracking systems, and in particular, to reduce a reliance upon prior-knowledge of the construction of the interventional device. One advantageous goal or target would be to facilitate or allow any interventional device to be used with any mapping system.

SUMMARY OF THE INVENTION

The invention is defined by the claims.

According to examples in accordance with an aspect of the invention, there is provided a processing system for determining a distance or distances between two or more device electrodes carried by an interventional device.

The processing system comprises: an electrode interface comprising a plurality of electrode connectors configured to connect to a plurality of electrodes, including at least the two or more device electrodes and two or more calibration electrodes; an electrode controller configured to, when the plurality of electrodes are connected to the electrode interface, apply, via the electrode interface, one or more electrical currents to the two or more calibration electrodes; and an electrode monitor.

The electrode monitor comprises: an input interface configured to, when the plurality of electrodes are connected to the electrode interface, obtain, via the electrode interface, electrical responses which include an electrical response of each device electrode to the one or more electrical currents applied to the two or more calibration electrodes; a processing unit, communicatively coupled to the input interface, configured to process at least the obtained electrical responses to determine the distance or distances between the two or more device electrodes; and optionally, an output interface communicatively coupled to the processing unit and configured to output any (and preferably all) determined distances.

Proposed approaches provide a mechanism for automatic determination of an inter-electrode distance. This information would significantly reduce a reliance upon prior-knowledge of the construction of the interventional device. In this way, relevant information for use in determining a mapping function (i.e. a voltage-to-position function) can be determined in advance, without the need for high accuracy physical measuring equipment or reliance upon information provided by device manufacturing. This can provide more accurate generation of a mapping function and ensure that the scale of any map or model constructed from monitored positions of the electrodes is correct.

The information generated by the processing unit thereby provides useful information about an interventional device, which can be used for improved tracking and/or monitoring of the location of the interventional device.

In some examples, the obtained electrical responses are electrical responses of the device electrodes when the device electrodes of the interventional device are positioned in a calibration chamber of a calibration device, wherein the calibration device is configured such that an electrical response of a device electrode to any of the one or more electrical currents changes responsive to a position of the device electrode within the calibration chamber.

Preferably, the calibration device comprises the two or more calibration electrodes. Preferably, the calibration device is configured such that an electric field generated between the calibration electrodes by the electrical current(s) applied to the calibration electrodes is substantially uniform.

In some examples, the calibration device further comprises a pair of sense electrodes a first predetermined distance apart from each other; the input interface is further configured to, when the pair of sense electrodes are connected to the electrode interface, obtain, via the electrode interface, the electrical response of each sense electrode to the one or more electrical currents applied to the two or more calibration electrodes; and the processing unit is configured to process at least the obtained electrical responses of the device electrodes and the sense electrodes to the one or more electrical currents applied to the two or more calibration electrodes and the first predetermined distance between the pair of sense electrodes to determine the distance or distances between the two or more device electrodes

In some embodiments, the electrode controller is configured to, when the plurality of calibration electrodes are connected to the electrode interface, apply, via the electrode interface, the one or more electrical currents to the two or more calibration electrodes to generate one or more electric fields between the calibration electrodes.

Each electrical response of the device electrodes and the sense electrodes may be a voltage response of the device or sense electrode to a generated electric field.

The processing unit may be configured to determine the distance between the two or more device electrodes by, for at least one pair of device electrodes: determining a first difference, being the difference between the electrical response of a first one of the pair of the device electrodes and the electrical response of a second, different one of the pair of device electrodes; determining a second difference, being the difference between the electrical response of a first one of the pair of sense electrodes and the electrical response of a second, different one of the pair of sense electrodes; dividing the first difference by the second difference to produce an intermediate result; and multiplying the absolute value of the intermediate result by the first predetermined distance to determine the distance between the pair of device electrodes.

In some examples, the two or more calibration electrodes comprise a pair of calibration electrodes that are a second predetermined distance apart from one another, the input interface is further configured to, obtain, via the electrode interface, the electrical response of each calibration electrode to the one or more electrical currents applied to the two or more calibration electrodes; and the processing unit is configured to process at least the obtained electrical responses of the device electrodes and the calibration electrodes to the one or more electrical currents applied to the two or more calibration electrodes and the known distance between the pair of calibration electrodes to determine the distance or distances between the two or more device electrodes.

In this way, the calibration electrodes may effectively act as the sense electrodes previously described. This approach provides a mechanism for determining the inter-electrode distances with a reduced number of electrodes. Use of dedicated sense electrodes is, however, more accurate, as the electric field between calibration electrodes may not be entirely uniform, e.g., due to a double capacitance effect.

Optionally, the processing unit is configured to determine the distance between the two or more device electrodes by, for at least one pair of device electrodes: determining a first difference, being the difference between the electrical response of a first one of the pair of the device electrodes and the electrical response of a second, different one of the pair of device electrodes; determining a third difference, being the difference between the electrical response of a first one of the pair of calibration electrodes and the electrical response of a second, different one of the pair of calibration electrodes; dividing the first difference by the third difference to produce a second intermediate result; and multiplying the absolute value of the second intermediate result by the second predetermined distance to determine the distance between the pair of device electrodes.

In some examples, each electrical response is a voltage response of a device electrodes to one or more of the electrical currents applied to the two or more calibration electrodes. However, other forms of electrical response, such as an impedance, could be used in alternative examples or embodiments.

In some examples, the input interface is further configured to, when the plurality of electrodes are connected to the electrode interface, monitor the electrical currents that are applied to the two or more calibration electrodes. The processing unit may be configured to process at least the obtained electrical responses and each monitored electrical current to determine the distance or distances between the two or more device electrodes.

There is also provided a calibration device for use with the processing system. The calibration device comprises a calibration chamber sized to house at least the portion of the interventional device carrying the device electrodes; and is configured such that an electrical response of a device electrode to any of the one or more electrical currents changes responsive to a position of the device electrode within the calibration chamber.

The calibration chamber of the calibration device is preferably filled with a conductive liquid. This improves the uniformity of an electric field between the calibration electrodes, and improves the accuracy of determining the inter-electrode distance.

In some examples, the calibration device comprises: an input channel, through which the interventional device moves to enter the calibration chamber; and an output channel, through which the interventional device moves to exit the calibration chamber, wherein the output channel is configured to be connectable to an introducer device for guiding the interventional device into a subject. This provides an approach for performing calibration as the interventional device is being inserted into the subject, and helps to ensure that the determination of inter-electrode distances is performed in a same or similar environment to the actual usage.

If the calibration chamber is filled with a conductive liquid, the conductive liquid may be blood from the subject to which the interventional device is to be inserted.

There is also proposed a system comprising the processing system herein described and the calibration device herein described.

Embodiments also provide a computer-implemented method for determining a distance or distances between two or more device electrodes carried by an interventional device, the computer-implemented method comprising: applying, using an electrode controller and via an electrode interface, one or more electrical currents to two or more calibration electrodes, wherein the electrode interface comprises a plurality of electrode connectors configured to connect to a plurality of electrodes, including at least the two or more device electrodes and the two or more calibration electrodes; obtaining, via the electrode interface and an input interface electrical responses which include an electrical response of each device electrode to the one or more electrical currents applied to the two or more calibration electrodes; processing, using a processing unit communicatively coupled to the input interface, at least the obtained electrical responses to determine the distance or distances between the two or more device electrodes; and optionally, outputting, via an output interface communicatively coupled to the processing unit, any (and preferably all) determined distances.

The skilled person would be readily capable of developing or modifying a method for performing the function of any herein described processing system.

There is also provided a computer program product comprising computer program code means which, when executed on a computing device having a processing system, cause the processing system to perform all of the steps of any herein claimed method.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 illustrates an interventional device system;
Figure 2 illustrates a system for determining an inter-electrode distance;
Figure 3 illustrates a calibration device for use in a system;
Figure 4 illustrates another calibration device for use in a system; and
Figure 5 illustrates a method according to an embodiment.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for determining a distance between electrodes on an interventional device. The electrical responses of the electrodes to electric current applied to calibration electrodes are used to derive or determine the inter-electrode distance(s).

Embodiments are based on the realization that it is possible to calibrate and determine inter-electrode distances based on the responses of the electrodes to an electrical current between calibration electrodes. This provides a simple and easy-to-use mechanism for determining inter-electrode distances.

Approaches may be employed in any circumstances where knowledge of a distance between electrodes is desirable or required, e.g., for performing a mapping of an anatomical cavity using device electrodes mounted on an interventional device.

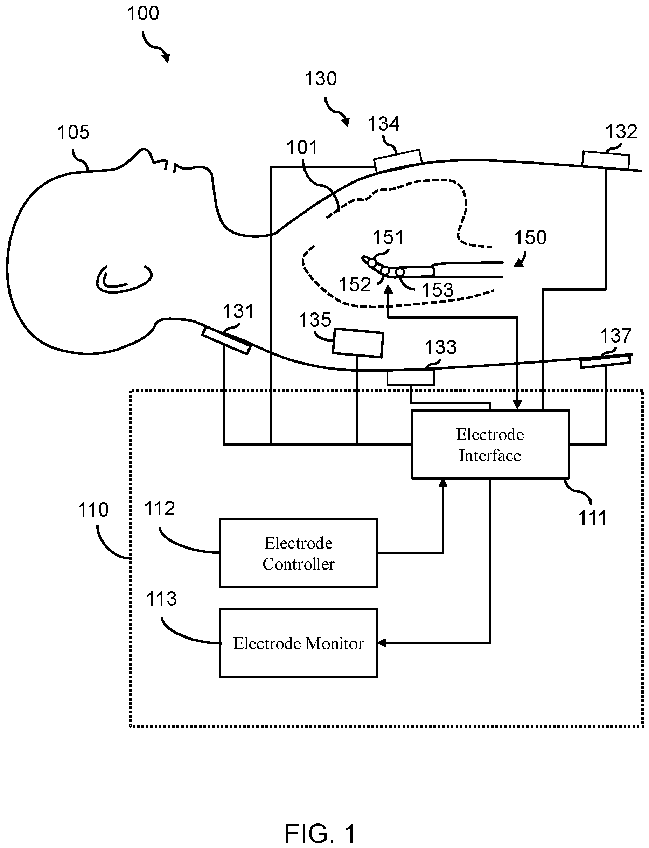

Figure 1 illustrates an interventional device system 100 for understanding a context of the invention.

The interventional device system 100 comprises an interventional device 150. The interventional device 150 is a device sized and configured to enter and move within an anatomical cavity 101 of a subject 105 or individual.

The interventional device system 100 also comprises a plurality of electrodes 131, 132, 133, 134, 135, 137, 151, 152, 153. Some of these electrodes 131, 132, 133, 134, 135, 137, form a set 130 of external electrodes that can be positioned on the exterior of the subject, and may be labelled external electrodes. Other electrodes 151, 152, 153 are carried by the interventional device 150, and may be labelled device electrodes or internal electrodes.

An electrode system 110 may control and/or monitor one or more electrical parameters at each electrode. The electrode system comprises an electrode interface 111 for connecting to each electrode (e.g. via a respective lead). An electrode controller 112 may control a current (or voltage) supplied to one or more of the electrodes. An electrode monitor 113 may monitor an electrical parameter or electrical response (e.g. current, voltage or impedance) with respect to one or more of the electrodes.

An example use for the electrodes of the interventional device system 100 is for a device tracking system. Electrical current provided to the set 130 of external electrodes by the electrode controller 112, via the electrode interface 111, may be configured to generate or induce crossing electric fields within the subject. The electrical response of one or more device electrodes 151, 152, 153 carried by the interventional device 150 to these fields can be used to track the relative location of the interventional device within the crossing electric fields.

More specifically, signals detected/sensed by the device electrodes 151, 152, 153 can be used to track the location of each device electrode and therefore the interventional device 150. The electrical response of each device electrode of the interventional device 150 to crossing electric fields induced within the subject can be monitored and mapped to a location in a predefined co-ordinate system (sometimes called an R-space) using an appropriate mapping function. This mapping function is used at least partly because induced electrical fields' distribution is inherently inhomogeneous due to the different dielectric properties and field absorption rates (related to conductivity) of the interrogated tissues.

Approaches for generating a mapping function are well established in the art. Example processes are disclosed by the European Patent Applications EP 0775466 A2, EP 3568068 A1 and EP 3607879 A1.

Generating a mapping function makes use of known inter-electrode distances to calibrate for the inhomogeneity in the crossing electric fields. The inter-electrode distance may refer to the geometric distance or the electrical distance (sometimes called the effective distance).

The tracked locations (e.g., in the predefined co-ordinate system) may be used to construct an anatomical model of the anatomical cavity in which the interventional device 150 travels. This is based on the assumption that the interventional device 150 will not be able to extend outside the inner surface of the anatomical cavity 101, so that points of a tracked location represent points inside the anatomical cavity. A cloud of points can be reconstructed into an anatomical model using any known system, such as those discussed in Berger, Matthew, et al. "A survey of surface reconstruction from point clouds." Computer Graphics Forum. Vol. 26. No. 1. 2017. Further mechanisms will be readily apparent to the skilled person.

Embodiments proposed by the present disclosure relate to mechanisms for automatic determination of an inter-electrode distance. This information would significantly reduce a reliance upon prior-knowledge of the construction of the interventional device, which may allow electrode systems 110 to be provided independently of knowledge of the interventional device 150.

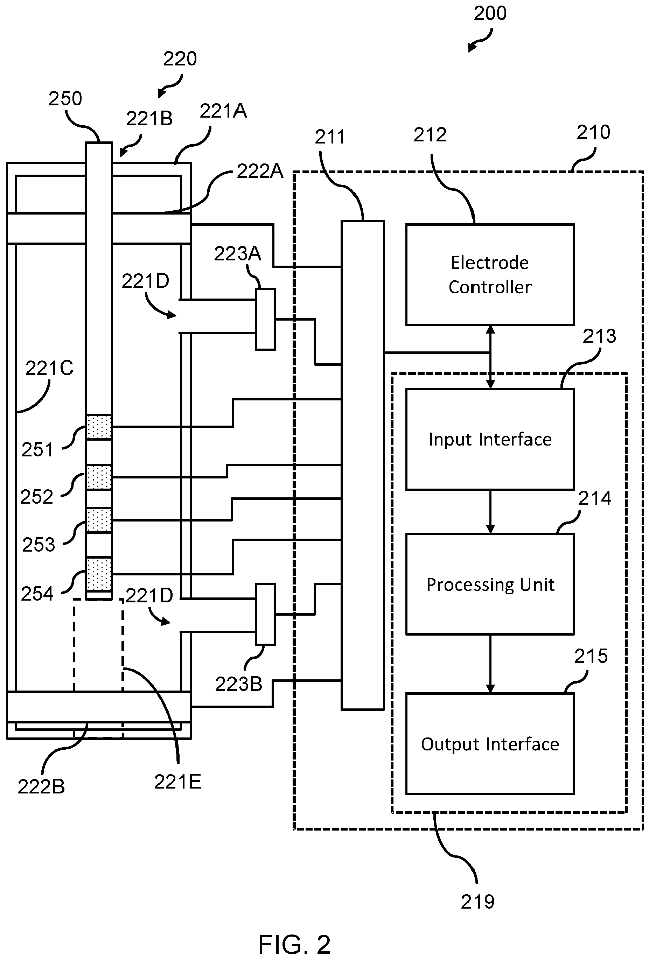

Figure 2 illustrates a system 200 for determining the inter-electrode distance between device electrodes 251, 252, 253, 254 carried by an interventional device 250.

The system 200 comprises a processing system 210 and a calibration device 220.

In this embodiment, the calibration device 220 comprises a (tubular) calibration chamber 221A that is sized to house the interventional device 250. Here, the calibration chamber is formed from a tube filled or fillable with a conductive liquid, such as water. The calibration chamber may comprise an input channel 221B that is sized to allow the interventional device to enter the calibration chamber.

The tube is preferably cylindrical, although this is not essential. For instance, the tube may be cuboidal.

When positioned inside the calibration chamber, the interventional device 250 is positioned at a particular position. Optionally, the calibration chamber 221A may comprise a pillar or support 221E for defining the position of the interventional device within the calibration chamber.

The calibration device 220 comprises a first calibration electrode 222A and a second calibration electrode 222B. The calibration electrodes 222A, 222B are positioned at either end of the calibration chamber 221. The calibration electrodes may be perforated or comprise one or more holes, e.g. to allow the passage of fluid.

The illustrated calibration device 220 also comprises a first sense electrode 223A and a second sense electrode 223B. The sense electrodes 223A, 223B are positioned a known distance apart from one another. The first sense electrode 223A is closer to the first calibration electrode 222A than the second sense electrode 223B. Similarly, the second sense electrode 223B is closer to the second calibration electrode 222B than the first sense electrode 223A.

Preferably, when located in the calibration chamber 221A, the interventional device 250 is positioned such that the device electrodes 251, 252, 253, 254 are between the sense electrodes 223A, 223B. The pillar or support 221E may be appropriately configured for this purpose. More preferably, when located in the calibration chamber 221A, the interventional device 250 is positioned such that the device electrodes are centered in the middle of the calibration chamber 221A.

The processing system 210 comprises an electrode interface 211, an electrode controller 212, an input interface 213, a processing unit 214 and (optionally) an output interface 215. The input interface 213, the processing unit 214 and (if present) the output interface 215 together form an electrode monitor 219.

The electrode interface 211 comprises a plurality of electrode connectors. Each electrode connector is configured to connect to a different one of the electrodes. In this way, each device electrode 251, 252, 253, 254, each calibration electrode 222A, 222B and each sense electrode 223A, 223B (if present) is connected to a respective electrode connector 221A.

The electrode controller 212 is configured to, when the plurality of electrodes are connected to the electrode interface, apply, via the electrode interface 211, electrical currents to the calibration electrodes.

In particular, the electrode controller is configured to drive the calibration electrodes with appropriate current(s) to create or induce an electric field between the first 222A and second 222B calibration electrodes. This may comprise, for instance, connecting a current source with a positive terminal connected to the first calibration electrode 222A (via the respective electrode connector) and a negative terminal connected to the second calibration electrode 222B. This approach creates a one-dimensional near-uniform current density between the calibration electrodes 222A, 222B (and therefore between the sense electrodes lying therebetween).

For improved accuracy, the inner surface 221C of the calibration chamber should be sufficient large such that there is little/negligible/no influence on the electric field at the position of the interventional device within the calibration chamber.

The sense electrodes may be connected to the calibration chamber 221A via fluid channels 221D, to reduce any influence on the electric field by the sense electrodes 223A, 223B.

The input channel 221B is configured so that, when the interventional device is positioned through the input channel 221B and an electric field is applied between the calibration electrodes 222A, 222B, the device electrodes lie in the direction of the electric field.

The input interface 213 is configured to, when the plurality of electrodes are connected to the electrode interface, obtain, via the electrode interface, electrical responses. The electrical responses include an electrical response of each device electrode to electrical current applied to the calibration electrodes and electrical responses of the sense electrodes.

Each electrical response may be a voltage response, e.g. a measured voltage at each electrode. The value of the voltage of each device and sensor electrode will change responsive to the position of the electrode within the electric field induced by supplying appropriate current to the calibration electrodes.

The second calibration electrode 222B may act as a reference electrode, so that measured electrical parameters are measured with respect to the same electrical parameter at the second calibration electrode 222B. Thus, a voltage response of an electrode may be a voltage difference between that electrode and the second calibration electrode 222B. Other variations would be readily apparent to the person skilled in the art. For instance, a voltage response of an electrode may be a voltage difference between that electrode and a ground or earth.

The processing unit 214 is communicatively coupled to the input interface 213. The processing unit 214 is configured to process at least the obtained electrical responses to determine the distance or distances between the two or more device electrodes.

In practice, the interventional device 250 can be inserted (via the input channel 221B) until the device electrodes 251, 252, 253, 254 are positioned in between the sensor electrodes 223A, 223B. This positioning can be identified by monitoring the voltages of each device and sensor electrode until the voltages at the device electrodes lie in between the voltages of the sensor electrodes.

The voltages of the device and sensor electrodes can be processed, together with the known distance L between the sense electrodes, to identify the distance between the device electrodes, i.e. determine the inter-electrode distance. Generally, an inter-electrode distance d between a first device electrode $C_1$ and a second device electrode $C_2$ (i.e. $d(C_2, C_1)$) can be defined as follows:

$$d(C_2, C_1) = L \left| \frac{V(C_2) - V(C_1)}{V(Sense_1) - V(Sense_2)} \right| \qquad (1)$$

where $V(C_2)$ represents the voltage at the second device electrode $C_2$, $V(C_1)$ represents the voltage at the first device electrode, $V(Sense_1)$ is the voltage at the first sense electrode 223A and $V(Sense_2)$ is the voltage at the second sense electrode 223B. L is the distance between the first and second sense electrodes.

Thus, determining an inter-electrode distance between a pair of device electrodes comprises:

determining a first difference ($V(C_2)$ - $V(C_1)$), being the difference between the electrical response of a first one of the pair of the device electrodes (i.e., the first device electrode) and the electrical response of a second, different one of the pair of device electrodes (i.e. the second device electrode);

determining a second difference ($V(Sense_1)$ - $V(Sense_2)$), being the difference between the electrical response of a first one of the pair of sense electrodes (i.e., the first sense electrode 223A) and the electrical response of a second, different one of the pair of sense electrodes (i.e., the second sense electrode 223B);

dividing the first difference by the second difference to produce an intermediate result; and

multiplying the absolute value of the intermediate result by the first predetermined distance to determine the distance between the pair of device electrodes.

In alternative examples, the dedicated sense electrodes 223A, 223B illustrated in Figure 2 may be omitted. Rather, the first 222A and second 222B calibration electrodes may also act as the sense electrodes. More specifically, the first calibration electrode 222A may act as the first sense electrode and the second calibration electrode 222B may act as the second sense electrode. Equation (1) may be modified accordingly, with L representing the distance between the first and second calibration electrodes, with $V(Sense_1)$ representing the voltage at the first calibration electrode and $V(Sense_2)$ representing the voltage at the second calibration electrode. This approach is less advantageous due to the uncontrollable impedance between the electrode surface and the fluid caused by air bubbles and polarization effects (the double layer capacitance).

In some examples, rather than detecting or determining the inter-electrode distance(s) using a voltage at each device electrode and each sense electrode, impedance measurements may be used instead. Mechanisms for determining an impedance are well known in the art.

Under such an approach, the inter-electrode distance $d(C_2, C_1)$ between a first device electrode $C_1$ and a second device electrode $C_2$ can be defined as follows:

$$d(C_2, C_1) = L \left| \frac{Z(C_2) - Z(C_1)}{Z(Sense)} \right| \tag{2}$$

in which $Z(C_1)$ represents the impedance between the first device electrode and the second sense electrode 223B, $Z(C_2)$ represents the impedance between the second device electrode and the second sense electrode 223B and $Z(Sense)$ represents the impedance between the first sense electrode 223A and the second sense electrode 223B. The value of $Z(Sense)$ is equivalent to determining the value of the impedance between the second sense electrode 223B and the second sense electrode 223B (which is zero) being subtracted from the impedance between the first sense electrode 223A and the second sense electrode 223B.

In some embodiments, a different reference point (i.e., rather than the second device electrode) is used. In this scenario, $Z(C_1)$ represents the impedance between the first device electrode and this reference point and $Z(C_2)$ represents the impedance between the second device electrode and this reference point. Of course, the value for $Z(Sense)$ in equation (2) could be replaced by ($Z(Sense_1)$ - $Z(Sense_2)$), where $Z(Sense_1)$ represents the impedance between the first sense electrode and this reference point and $Z(Sense_2)$ represents the impedance between the second sense electrode and this reference point.

Using the approaches described with reference to Figure 2: the calculated inter-electrode distances are independent of the specific impedance (conductivity, permittivity) of the fluid.

Moreover, for the approach using at least equation (1), the impedance of the excitation source (i.e., the calibration electrodes) is not relevant, because the distances are calculated from the voltage division with respect to the voltage difference between the sense electrodes. Thus, the driving of the calibration electrodes to generate the electric field can be performed using a voltage source, a current source or a combination. However, for improved safety, the calibration electrodes may be driven using a power source with sufficient series resistance to limit the maximum current. For instance, the electrode controller may comprise a 100V voltage source that drives the calibration electrode(s) with a high series resistance (e.g. >100kΩ) to limit the maximum current, e.g., to 1mA. Of course, other resistances may be chosen depending on a desired maximum current and/or voltage of the voltage source.

For the above-described mechanism, the electrode controller generates one excitation current (between the calibration electrodes) and the input interface measures at least four voltages (at two sense electrodes and each of the at least two device electrode).

Approaches may be repeated for each pair of device electrodes for which an inter-electrode distance is desired or required.

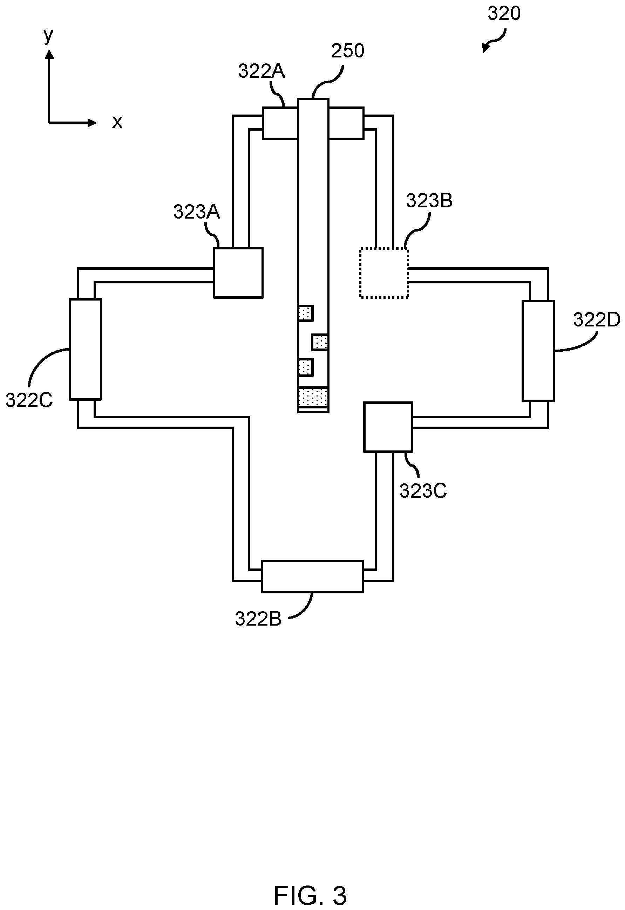

Figure 3 illustrates a modified version of a calibration device 320 for a system.

The calibration device differs in that two electric fields can be generated using two pairs of calibration electrode. The generated electric fields are orthogonal to one another. This approach facilitates determination of distances between device electrodes in two dimensions (e.g. rather than just a single dimension) - e.g. in an x-dimension and a y-dimension.

Thus, the calibration device 320 comprises a first calibration electrode 322A, a second calibration electrode 322B, a third calibration electrode 322C, and a fourth calibration electrode 322D.

The electrode controller is configured, to when the plurality of electrodes are connected to the electrode interface, drive the calibration electrodes with appropriate current(s) to create or induce a first electric field between the first 322A and second 322B calibration electrodes (in a y-dimension) and create or induce a second electric field between the third 322C and fourth 322D calibration electrodes (in an x-dimension). The calibration electrodes are positioned so that the first and second electric fields are orthogonal with respect to one another (i.e. are perpendicular to one another). In this way, each electric field defines (and lies in) a different dimension in which inter-electrode distances can be calculated.

The generated electric fields may be of different frequencies, e.g. so that it is possible to distinguish the voltage response of an electrode to each electric field separately (through appropriate filtering). Alternatively, a time-multiplexing approach could be used to facilitate identification of the response of electrodes to different electric fields.

The mechanism for determining an inter-electrode distance between different device electrodes for each dimension may employ any previously described approach, e.g. by applying any of equations (1) or (2). In particular, a voltage and/or current used to derive electrical parameter(s) for use in equation (1) or (2) may be a voltage and/or current for a particular electric field (e.g. at a particular frequency or point in time). A distance in a particular dimension can be established using the voltage or impedances for the particular electric field lying in that dimension.

In this way, an inter-electrode distance in an x-dimension and a distance in a y-dimension can be determined.

The sense electrodes must be distanced from one another in both directions/dimensions, e.g. to create an x-distance and a y-distance.

In some examples, the sense electrodes comprises at least three sense electrodes 323A, 323B, 323C, with a first pair of sense electrodes 323A, 323B lying in a direction of the first electric field and a second pair of sense electrodes 323B, 323C lying in a direction of a second electric field. A distance between the first pair of sense electrodes may represent the distance for the first dimension and a distance between the second pair of sense electrodes may represent the distance for the second dimension.

In another example, the sense electrodes comprises only two sense electrodes 323A, 323C. A relative distance in a first dimension represents an x-distance and a relative distance in a second dimension represents a y-distance.

The electrode(s) of the calibration device 320 may be coupled to the processing system, previously described with reference to Figure 2. The electrode controller of the processing system may be configured to drive the calibration electrode(s) to generate two orthogonal electric fields.

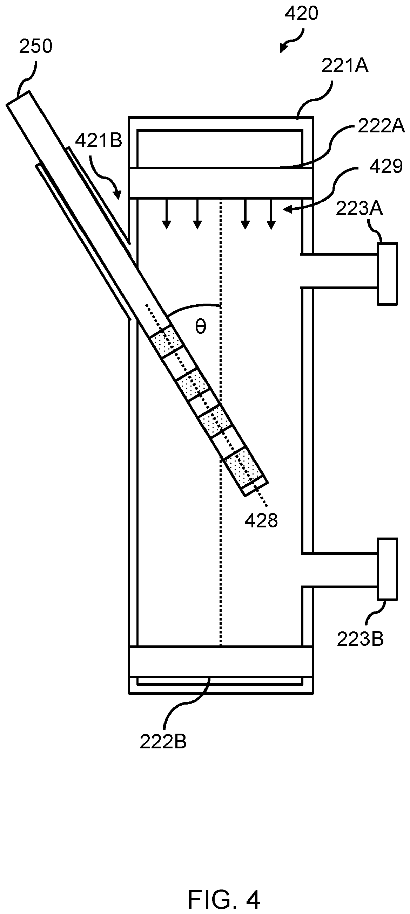

Figure 4 illustrates another variant of a calibration device 420 for a system. This approach is used to calibrate inter-electrode distances for an obliquely incident field with respect to the length axis of the interventional device.

The calibration device 420 differs from the calibration device 220 in that the input channel 421B of the calibration device 420 is configured so that, when the interventional device is positioned through the input channel 421B and an electric field is applied between the calibration electrodes 222A, 222B, the device electrodes lie in along a hypothetical line 428 that is angled or inclined with respect to the direction 429 of the electric field.

The hypothetical line 428 makes a defined angle $\theta$ with respect to the direction 429 of the electric field induced between the calibration electrodes. Any above-described approach for determining a distance or distances between device electrodes may be applied to determine a distance between the device electrodes, excepting that any determined distances should be further divided by $\cos(\theta)$ to calculate the distances between the electrodes of the interventional device.

Use of a system in which the input channel 421B is positioned so that the interventional device is angled/inclined with respect to the electric field induced between the calibration electrodes can advantageously reduce a size of the calibration device and increase a uniformity of the electric field, as the interventional device will create less interference with the electric field as it no longer passes through the calibration electrode(s).

In any above-described embodiments, deviations in the voltages across the calibration electrodes and the sense electrodes can be used as a quality measure. For example, a calibration voltage (a voltage between the calibration electrodes) that is too high is indicative of air bubbles in the tube, while a sense voltage (a voltage between the sense electrodes) that is too low can be the result of insufficient liquid surrounding one or more of the sense electrodes.

A "too high" calibration voltage may be a voltage that is no less 5% or 10% greater than the average voltage for a given conductivity and applied current between the calibration electrodes. A "too low" sensed voltage may be a voltage that is no more than 1% or 5% less than the average voltage for a given conductivity and applied current between the calibration electrodes.

Similarly, impedance values that differ from an average by more than predetermined amounts can indicate air bubbles or dirt on a surface. The term "voltage" may be replaced by the term "impedance" in the preceding paragraphs to exploit this recognition.

For a number of techniques that make use of the electrodes of the interventional device, it is useful to know the impedance of the device electrodes, as well as other characteristics such as temperature dependencies. Moreover, the impedance and crosstalk of the wiring is frequency dependent and thus relevant to characterize or identify. Characterization of this data can be performed by making additional measurements that can be with the calibration device, e.g. by also measuring the impedances of the device electrodes. One of the calibration and/or sense electrodes can serve as a reference.

These measurements can be performed with the calibration chamber being filled with blood (e.g. of the subject to be investigated), but alternative liquids such as saline could act as a proxy. Temperature dependencies can also be easily determined, for example by placing the calibration device in an oven or using heated liquid to fill the calibration chamber.

In some examples, any herein described calibration unit may comprise an output channel, being a channel through which the interventional device moves to exit the calibration chamber. The output channel may be configured to be connectable to an introducer device for guiding the interventional device into a subject.

In this way, the calibration unit can effectively be used to perform calibration as the interventional device is being inserted into the subject. This advantageously ensures that the determination of inter-electrode distances is performed in a same or similar environment to the actual usage.

In such examples, the conductive liquid filling the calibration chamber may, for instance, comprise blood from the subject (e.g., passed to the calibration chamber via the introducer device. This improves the accuracy and relevance of any calculated inter-electrode distance.

Embodiments make use of a processing unit.

The processing unit can be implemented in numerous ways, with software and/or hardware, to perform the various functions required. A processor is one example of a processing unit which employs one or more microprocessors that may be programmed using software (e.g., microcode) to perform the required functions. A processing unit may however be implemented with or without employing a processor, and also may be implemented as a combination of dedicated hardware to perform some functions and a processor (e.g., one or more programmed microprocessors and associated circuitry) to perform other functions.

Examples of processing unit components that may be employed in various embodiments of the present disclosure include, but are not limited to, conventional microprocessors, application specific integrated circuits (ASICs), and field-programmable gate arrays (FPGAs).

In various implementations, a processor or processing unit may be associated with one or more storage media such as volatile and non-volatile computer memory such as RAM, PROM, EPROM, and EEPROM. The storage media may be encoded with one or more programs that, when executed on one or more processors and/or processing units, perform the required functions. Various storage media may be fixed within a processor or processing unit or may be transportable, such that the one or more programs stored thereon can be loaded into a processor or processing unit.

The skilled person would be readily capable of developing a method for performing the function of any herein described processing system. Thus, each module of the processing system (e.g., the input interface, the processing unit and the optional output interface) may perform a different process or set of one or more steps for a method.

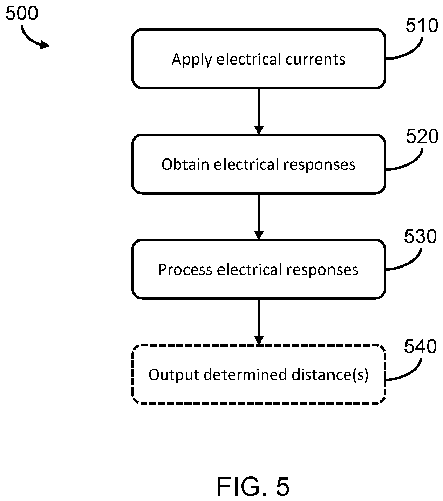

For the sake of completeness, Figure 5 illustrates a method 500 according to an embodiment.

The method 500 comprises a step 510 of applying, using an electrode controller and via an electrode interface, one or more electrical currents to two or more calibration electrodes, wherein the electrode interface comprises a plurality of electrode connectors configured to connect to a plurality of electrodes, including at least the two or more device electrodes and the two or more calibration electrodes.

The method 500 also comprises a step 520 of obtaining, via the electrode interface and an input interface electrical responses which include an electrical response of each device electrode to the one or more electrical currents applied to the two or more calibration electrodes.

The method 500 also comprises a step 530 of processing, using a processing unit communicatively coupled to the input interface, at least the obtained electrical responses to determine the distance or distances between the two or more device electrodes.

The method optionally comprises a step 540 of outputting, via an output interface communicatively coupled to the processing unit, any (and preferably all) determined distances.

Approaches for performing the processing of the obtained electrical responses have been previously described.

It will be understood that disclosed methods are preferably computer-implemented methods. As such, there is also proposed the concept of a computer program comprising code means for implementing any described method when said program is run on a processing unit, such as a computer. Thus, different portions, lines or blocks of code of a computer program according to an embodiment may be executed by a processing unit or computer to perform any herein described method. In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

## Claims

1. A processing system (210) for determining a distance or distances between two or more device electrodes (251, 252, 253) carried by an interventional device (250), the processing system comprising:

    an electrode interface (211) comprising a plurality of electrode connectors configured to connect to a plurality of electrodes, including at least the two or more device electrodes and two or more calibration electrodes (222A, 222B, 322A, 322B, 322C, 322D);
    an electrode controller (212) configured to, when the plurality of electrodes are connected to the electrode interface, apply, via the electrode interface, one or more electrical currents to the two or more calibration electrodes; and
    an electrode monitor (219) comprising:

       an input interface (213) configured to, when the plurality of electrodes are connected to the electrode interface, obtain, via the electrode interface, electrical responses which include an electrical response of each device electrode to the one or more electrical currents applied to the two or more calibration electrodes;
       a processing unit (214), communicatively coupled to the input interface, configured to process at least the obtained electrical responses to determine the distance or distances between the two or more device electrodes; and
       optionally, an output interface (215) communicatively coupled to the processing unit and configured to output any determined distances.

2. The processing system of claim 1, wherein the obtained electrical responses are electrical responses of the device electrodes when the device electrodes of the interventional device are positioned in a calibration chamber (221A) of a calibration device (220, 320, 420),
    wherein the calibration device is configured such that an electrical response of a device electrode to any of the one or more electrical currents changes responsive to a position of the device electrode within the calibration chamber.

3. The processing system of claim 2, wherein the calibration device comprises the two or more calibration electrodes.

4. The processing system of claim 3, wherein the calibration device further comprises a pair of sense electrodes (223A, 223B, 323A, 323B, 323C) a first predetermined distance apart from each other;

    the input interface is further configured to, when the pair of sense electrodes are connected to the electrode interface, obtain, via the electrode interface, the electrical response of each sense electrode to the one or more electrical currents applied to the two or more calibration electrodes; and
    the processing unit is configured to process at least the obtained electrical responses of the device electrodes and the sense electrodes to the one or more electrical currents applied to the two or more calibration electrodes and the first predetermined distance between the pair of sense electrodes to determine the distance or distances between the two or more device electrodes

5. The processing system of claim 4, wherein:

   the electrode controller is configured to, when the plurality of calibration electrodes are connected to the electrode interface, apply, via the electrode interface, the one or more electrical currents to the two or more calibration electrodes to generate one or more electric fields between the calibration electrodes; and
   each electrical response of the device electrodes and the sense electrodes is a voltage response of the device or sense electrode to a generated electric field.

6. The processing system of claim 4 or 5, wherein the processing unit is configured to determine the distance between the two or more device electrodes by, for at least one pair of device electrodes:

   determining a first difference, being the difference between the electrical response of a first one of the pair of the device electrodes and the electrical response of a second, different one of the pair of device electrodes;
   determining a second difference, being the difference between the electrical response of a first one of the pair of sense electrodes and the electrical response of a second, different one of the pair of sense electrodes;
   dividing the first difference by the second difference to produce an intermediate result; and
   multiplying the absolute value of the intermediate result by the first predetermined distance to determine the distance between the pair of device electrodes.

7. The processing system of claim 3, wherein:

   the two or more calibration electrodes comprise a pair of calibration electrodes that are a second predetermined distance apart from one another,
   the input interface is further configured to, obtain, via the electrode interface, the electrical response of each calibration electrode to the one or more electrical currents applied to the two or more calibration electrodes; and
   the processing unit is configured to process at least the obtained electrical responses of the device electrodes and the calibration electrodes to the one or more electrical currents applied to the two or more calibration electrodes and the known distance between the pair of calibration electrodes to determine the distance or distances between the two or more device electrodes

8. The processing system of claim 7, wherein the processing unit is configured to determine the distance between the two or more device electrodes by, for at least one pair of device electrodes:

   determining a first difference, being the difference between the electrical response of a first one of the pair of the device electrodes and the electrical response of a second, different one of the pair of device electrodes;
   determining a third difference, being the difference between the electrical response of a first one of the pair of calibration electrodes and the electrical response of a second, different one of the pair of calibration electrodes;
   dividing the first difference by the third difference to produce a second intermediate result; and
   multiplying the absolute value of the second intermediate result by the second predetermined distance to determine the distance between the pair of device electrodes.

9. The processing system of any of claims 1 to 8, wherein each electrical response is a voltage response of a device electrodes to one or more of the electrical currents applied to the two or more calibration electrodes.

10. The processing system of claim 9, wherein:

    the input interface is further configured to, when the plurality of electrodes are connected to the electrode interface, monitor the electrical currents that are applied to the two or more calibration electrodes; and
    the processing unit is configured to process at least the obtained electrical responses and each monitored electrical current to determine the distance or distances between the two or more device electrodes.

11. A calibration device (220, 320, 420) for use with the processing system of any of claims 2 to 10, wherein the calibration device:

    comprises a calibration chamber sized to house at least the portion of the interventional device carrying the device electrodes; and
    is configured such that an electrical response of a device electrode to any of the one or more electrical currents changes responsive to a position of the device electrode within the calibration chamber.

**12.** The calibration device of claim 11, wherein the calibration device comprises:

an input channel, through which the interventional device moves to enter the calibration chamber; and
an output channel, through which the interventional device moves to exit the calibration chamber, wherein the output channel is configured to be connectable to an introducer device for guiding the interventional device into a subject.

**13.** A system (200) comprising:

the processing system (210) of any of claims 2 to 10; and
the calibration device (220, 320, 420) of any of claims 11 to 12.

**14.** A computer-implemented method (500) for determining a distance or distances between two or more device electrodes (251, 252, 253, 254) carried by an interventional device (250), the computer-implemented method comprising:

applying (510), using an electrode controller (212) and via an electrode interface (211), one or more electrical currents to two or more calibration electrodes (222A, 222B, 322A, 322B, 322C, 322D), wherein the electrode interface comprises a plurality of electrode connectors configured to connect to a plurality of electrodes, including at least the two or more device electrodes and the two or more calibration electrodes;
obtaining (520), via the electrode interface and an input interface (213), electrical responses which include an electrical response of each device electrode to the one or more electrical currents applied to the two or more calibration electrodes;
processing (530), using a processing unit (214) communicatively coupled to the input interface, at least the obtained electrical responses to determine the distance or distances between the two or more device electrodes; and
optionally, outputting (540), via an output interface (215) communicatively coupled to the processing unit, any determined distances.

**15.** A computer program product comprising computer program code means which, when executed on a computing device having a processing system, cause the processing system to perform all of the steps of the method according to claim 14.

FIG. 1

FIG. 2

FIG. 3

FIG. 4

500

510
Apply electrical currents

520
Obtain electrical responses

530
Process electrical responses

540
Output determined distance(s)

FIG. 5

Europäisches
Patentamt

European
Patent Office

Office européen
des brevets

# EUROPEAN SEARCH REPORT

Application Number

EP 22 16 5745

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/052331 A1 (BEN-HAIM SHLOMO [IT]) 25 February 2021 (2021-02-25) * abstract; figure 1 * * paragraph [0097] – paragraph [0099] * * paragraph [0103] * * paragraph [0118] * | 1-15 | INV. A61B5/06 A61B5/287 A61B5/00  ADD. A61B34/20 |
| A | WO 2020/247619 A1 (ACUTUS MEDICAL INC [US]) 10 December 2020 (2020-12-10) * abstract * | 1-15 | |
| A | WO 2021/048420 A1 (NAVIX INT LTD; BEN HAIM SHLOMO [IT]; DICHTERMAN ELI [IL]) 18 March 2021 (2021-03-18) * abstract * | 1-15 | |
| A | US 8 543 188 B2 (VON JAKO RON ANDREW [US]; GROSZMANN DANIEL EDUARDO [US] ET AL.) 24 September 2013 (2013-09-24) * abstract * | 1-15 | |

TECHNICAL FIELDS
SEARCHED (IPC)

A61B

The present search report has been drawn up for all claims

| Place of search | Date of completion of the search | Examiner |
|---|---|---|
| The Hague | 19 August 2022 | Dhondt, Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 5745

This annex lists the patent family members relating to the patent documents cited in the above-mentioned European search report.
The members are as contained in the European Patent Office EDP file on
The European Patent Office is in no way liable for these particulars which are merely given for the purpose of information.

19-08-2022

| Patent document cited in search report | | Publication date | Patent family member(s) | | Publication date |
|---|---|---|---|---|---|
| US 2021052331 | A1 | 25-02-2021 | CN | 112367907 A | 12-02-2021 |
| | | | EP | 3790453 A1 | 17-03-2021 |
| | | | US | 2021052331 A1 | 25-02-2021 |
| | | | WO | 2019215574 A1 | 14-11-2019 |
| WO 2020247619 | A1 | 10-12-2020 | AU | 2020287341 A1 | 25-11-2021 |
| | | | CA | 3135773 A1 | 10-12-2020 |
| | | | CN | 114072049 A | 18-02-2022 |
| | | | EP | 3979906 A1 | 13-04-2022 |
| | | | IL | 287822 A | 01-01-2022 |
| | | | JP | 2022535134 A | 04-08-2022 |
| | | | US | 2022226046 A1 | 21-07-2022 |
| | | | WO | 2020247619 A1 | 10-12-2020 |
| WO 2021048420 | A1 | 18-03-2021 | NONE | | |
| US 8543188 | B2 | 24-09-2013 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0775466 A2
- EP 3568068 A1
- EP 3607879 A1

**Non-patent literature cited in the description**

- **BERGER, MATTHEW et al.** A survey of surface reconstruction from point clouds. *Computer Graphics Forum,* 2017, vol. 26 (1